# EUROPÄISCHE PATENTSCHRIFT

(11) **EP 0 897 710 B1**
(45) Veröffentlichungstag und Bekanntmachung des Hinweises auf die Patenterteilung: **24.11.2004**
(21) Anmeldenummer: 98115822.3
(22) Anmeldetag: 21.08.1998
(51) Int. Cl.: A61K 6/087, C08G 59/68, C08G 65/18

(54) **Lichtinduziert kationisch härtende Zusammensetzungen und deren Verwendung**
Light-initiated cationic curable compositions and their use
Compositions durcissables par réaction cationique initiée par la lumière et leur utilisation

(30) Priorität: 21.08.1997 DE 19736471
(43) Veröffentlichungstag der Anmeldung: 24.02.1999
(73) Patentinhaber: 3M ESPE AG, 82229 Seefeld (DE)
(72) Erfinder: Weinmann, Wolfgang, 82205 Gilching (DE); Eckhardt, Gunther, 82346 Frieding (DE)

(56) Entgegenhaltungen:
- EP-A- 0 728 790
- EP-A- 0 764 691
- WO-A-96/13538
- WO-A-97/18792
- WO-A-98/47046
- BI, YUBAI ET AL: "A Visible Light Initiating System for Free Radical Promoted Cationic Polymerization" MACROMOLECULES (1994), 27(14), 3683-93 , 1994, XP000457200

## Beschreibung

Die Erfindung betrifft Zusammensetzungen auf der Basis von Epoxidharzen und bzw. oder Oxetanen, die während oder nach der Bestrahlung mit sichtbarem Licht durch kationische Polymerisation aushärten. Insbesondere betrifft die Erfindung Zusammensetzungen mit einer nur geringen Eigenfarbe und deren Verwendung in dentalen Präparaten.

Bekanntermaßen können Zusammensetzungen mit Verbindungen, die Epoxid- und bzw. oder Oxetangruppen enthalten, kationisch aushärten. Die Auslösung der kationischen Polymerisation erfolgt üblicherweise durch LEWIS- oder BRÖNSTED-Säuren, wobei diese Säuren entweder der kationisch härtbaren Zubereitung zugesetzt oder durch vorgelagerte chemische und insbesondere photochemische Reaktionen erzeugt werden können.

So sind für epoxidgruppenhaltige Zusammensetzungen eine Reihe von sogenannten Photoinitiatoren bekannt, die unter Einwirkung von Licht des Wellenlängenbereiches von 215 bis 400 nm unter Bildung von BRÖNSTED-Säuren zerfallen. Zu diesen Initiatoren zählen beispielsweise Diazoniumverbindungen (US-A-3 205 157), Sulfoniumverbindungen (US-A-4 173 476) und lodoniumverbindungen (US-A-4 264 703, US-A-4 394 403). Bei den erwähnten Beispielen ist man für die Polymerisation kationisch härtbarer Massen allerdings auf die Verwendung von ultraviolettem Licht angewiesen.

Es sind auch photolabile Substanzen bekannt, welche durch Bestrahlung mit sichtbarem Licht LEWIS- bzw. BRÖNSTED-Säuren freisetzen, die die Polymerisation der kationisch härtbaren Zubereitungen bewirken können. Bei diesen Photoinitiatoren handelt es sich im allgemeinen um Derivate der Cyclopentadienyl-Eisen-Aren-Komplexe (EP-A-0 094 915, WO 96/03453, EP-A-0 661 324). Diese Photoinitiatoren haben den Nachteil, braune bis schwarze Polymerisate zu ergeben, was in den Fällen dentaler Anwendungen zu ästhetisch unbefriedigenden Ergebnissen führt. Außerdem tritt bei der Aushärtung ein intensiver Geruch nach Isopropylbenzol auf, was in dentalen Anwendungen unerwünscht ist.

Weiterhin sind Initiatorsysteme bekannt, die im sichtbaren Bereich kationische Polymerisation ermöglichen. Diese enthalten jedoch farbige Sensibilisatoren, beispielsweise Xanthene oder Fluorene, deren chromophore Gruppen erhalten bleiben und die Polymerisate bunt färben (WO-95/14716; Chemical Abstracts, Vol. 121, 1994, Ref. 58043z) und daher nicht zur Verwendung ästhetisch einwandfreier dentaler Massen geeignet sind.

Gemäß der WO 96/13538 werden im sichtbaren Licht härtbare Epoxidsysteme mit verbesserter Härtungstiefe beschrieben, die aus
a) einem kationisch polymerisierbaren Epoxidharz,
b) einem Hydroxylgruppen-enthaltendem Material,
c) einem Aryliodoniumsalz und
d) einer alpha-Dicarbonylverbindung
bestehen. Die alpha-Dicarbonylverbindung wirkt als Sensibilisator im sichtbaren Bereich, wobei besonders bevorzugt das Campherchinon eingesetzt wird. Bekanntlich zerfällt Campherchinon bei Bestrahlung mit sichtbarem Licht unter Bildung freier Radikale, was seit langer Zeit zur Initiierung des Aushärtevorganges von doppelbindungshaltigen Zubereitungen und bevorzugt von dentalen Präparaten genutzt wird.

Weiterhin sind kombinierte Initiatorsysteme aus Campherchinon und Iodoniumverbindungen bekannt, die jedoch nur zur Polymerisation doppelbindungshaltiger oder anderer radikalisch härtender Massen oder zur Polymerisation von Hybrid-Monomermischungen eingesetzt werden (US-A-5 545 676, US-A-4 828 583).

Die Verwendung von Hydroxylgruppen-haltigen Verbindungen führt zwar zu der bereits aus vielen Publikationen bekannten Reaktionsbeschleunigung bei der kationischen Polymerisation von Epoxyverbindungen, initiiert durch lodoniumverbindungen, und ist bereits zur Erzielung flexibilisierter Epoxidmassen beschrieben (US-A-4 256 828; US-A-4 231 951; EP-B-0 119 425; DE-A-4 324 322.3), jedoch kann die Verwendung von niedermolekularen Hydroxylgruppen-haltigen Verbindungen in relativ hohen Konzentrationen, wie sie in den Patentbeispielen 1 bis 6, 10 bis 16, 22, 23, 32 und 33 der WO 96/13538 angegeben sind, zu einer unvollständigen Einbindung in das polymere Netzwerk mit den Folgen schlechter mechanischer Eigenschaften und hoher extrahierbarer Anteile führen.

Aufgabe der Erfindung ist es, lichtinduziert, kationisch härtende Zusammensetzungen bereitzustellen, die eine geringe Eigenfarbe besitzen, die geruchsarm aushärten und deren Aushärtung zu Massen mit sehr guten mechanischen Eigenschaften, wie hohe Kohäsion, hohe Druckfestigkeit und Biegefestigkeit und geringen extrahierbaren Anteilen führt.

Diese Aufgabe wird gelöst durch lichtinduziert, kationisch härtende Zusammensetzungen, enthaltend
a) 0,01 bis 8 Gew.-%, bevorzugt 0,1 bis 5 Gew.-% mindestens einer Diaryliodoniumverbindung oder eines Gemisches von Diaryliodoniumverbindungen der folgenden Struktur:

   [((R¹)ₐAr¹)-I-(Ar²(R²)_{b})]⁺ Y⁻

   wobei bedeuten:
   - Ar¹ , Ar²: unabhängig voneinander verschiedene, substituierte oder unsubstituierte, kondensierte oder nichtkondensierte aromatische Systeme mit 4 bis 20 C-Atomen, vorzugsweise Phenyl, Tolyl, Cumyl, Anisyl, Chlorphenyl, Nitrophenyl, Naphtyl, Thienyl, Furanyl und Pyrazolyl
   - R¹, R²: unabhängig voneinander ein H-Atom, einen aliphatischen Rest mit 1 bis 19, vorzugsweise 1 bis 9 C-Atomen, wobei ein oder mehrere C-Atome durch O, C = O,-O(C = O)-, F, Cl, Br, SiR³₃ und/oder NR³₂ ersetzt sein können, wobei R³ ein aliphatischer Rest mit 1 bis 7 C-Atomen ist, bei dem ein oder mehrere C-Atome durch O, C=O und/oder -O(C=O)- ersetzt sein können, wobei die Aromaten Ar¹ und Ar² über R¹ und/oder R² miteinander verbunden sein können,
   - a und b: unabhängig voneinander eine ganze Zahl von 1 bis 5, und
   - Y⁻: ein wenig nukleophiles Anion der Formel

   KₓL_{y}⁻
   worin bedeuten:
   - K: ein Element der III., V. oder VII. Hauptgruppe, vorzugsweise B, Al, P, Sb, As oder I
   - x: eine Zahl von 1 bis 4
   - L: voneinander unabhängige aromatische, araliphatische oder cycloaliphatische Reste mit bis zu 25 C-Atomen, bei denen ein oder mehrere C-Atome durch F, Cl, Br oder I ersetzt sein können, und
   - y: eine Zahl von 0 bis 6,
b) 0,01 bis 8 Gew.%, bevorzugt 0,1 bis 5 Gew.% mindestens einer α-Dicarbonylverbindung,
c) 10,0 bis 99,9 Gew.% mindestens einer Epoxidgruppen- und/oder Oxetangruppen-enthaltenden Verbindung,
d) 0 bis 85 Gew.% an Modifikatoren, wie Füllstoffen, Farbstoffen, Pigmenten, Fließverbesserern, Thixotropiemitteln, polymeren Verdickem, oxidierend wirkenden Zusatzstoffen, Stabilisatoren und Verzögerern,
   dadurch gekennzeichnet, daß sie zusätzlich
e) 0,001 bis 5 Gew.-%, bevorzugt 0,01 bis 3 Gew.-% mindestens eines aromatischen Amins,
enthalten.

Es ist überraschend, daß die eingesetzten Amine des Bestandteils e) eine beschleunigende Wirkung auf die lichtinduzierte kationische Polymerisation haben. So ist im Stand der Technik beschrieben (DE-A-195 34 594, WO 96/13538), daß sich Amine verzögernd oder sogar inhibierend auf die Polymerisation auswirken. Völlig überraschend ist zudem, daß ein schon sehr geringer Zusatz, beispielsweise 0,001 Gew.-% in der polymerisierenden Zusammensetzung beschleunigende Wirkung hat.

Die Diaryliodoniumverbindungen des Bestandteils a) weisen die folgende Struktur auf:

[((R¹)ₐAr¹)-I-(Ar²(R²)_{b})]⁺ Y⁻

Ar¹ und Ar² können unabhängig voneinander verschiedene, substituierte oder unsubstituierte, kondensierte oder nichtkondensierte aromatische Systeme mit 4 bis 20 C-Atomen sein, wie beispielsweise Phenyl, Tolyl, Cumyl, Anisyl,Chlorphenyl, Nitrophenyl, Naphtyl, Thienyl, Furanyl und Pyrazolyl, wobei R¹ und R² gleich oder verschieden sind und unabhängig voneinander ein H-Atom, einen aliphatischen Rest mit 1 bis 19, vorzugsweise 1 bis 9 C-Atomen, wobei ein oder mehrere C-Atome durch O, C=O, -O(C=O)-, F, Cl, Br, SiR³₃ und/oder NR³₂ ersetzt sein können, wobei R³ ein aliphatischer Rest mit 1 bis 7 C-Atomen ist, bei dem ein oder mehrere C-Atome durch O, C=O und/oder -O(C=O)- ersetzt sein können, bedeuten und a und b unabhängig voneinander 1 - 5 sein können. Die Aromaten Ar¹ und Ar² können über R¹ und/oder R² miteinander verbunden sein.

Das Gegenanion Y⁻ ist ein wenig nucleophiles Anion der folgenden Struktur

KₓL_{y}⁻

wobei K ein Element der III., V. oder VII. Hauptgruppe, wie beispielsweise B, Al, P, Sb, As oder I ist und x Zahlenwerte von 1 bis 4 annehmen kann. L bedeutet unabhängig voneinander aromatische, araliphatische oder cycloaliphatische Reste mit bis zu 25 C-Atomen, bei denen ein oder mehrere C-Atome durch F, Cl, Br oder ersetzt sein können, und y kann Zahlenwerte von 0 bis 6 annehmen. Bevorzugte Reste L sind Pentafluorphenyl-, Tetrafluorphenyl-, Trifluorphenyl-, Fluorphenyl-, Phenyl-, 4-Trifluormethylphenyl-, 3,5-Bis(trifluormethyl)phenyl- und 2,4,6-Tris(trifluormethyl)phenyl-. Ein besonders bevorzugtes Gegenanion Y⁻ ist B(C₆F₅)₄⁻. Weitere Diaryliodoniumverbindungen sind beispielsweise auch in der US-A-4 264 703 beschrieben.

Besonders geeignete Diaryliodoniumverbindungen sind:
- Diphenyliodoniumtetrakis(pentafluorophenyl)borat
- Bis-(4-methylphenyl)iodoniumtetrakis(pentafluorophenyl)borat
- Phenyl-4-methylphenytiodoniumtetrakis(pentafluorophenyl)borat
- Phenyl-4-methoxyphenyliodoniumtetrakis(pentafluorophenyt)borat
- Phenyl-3-nitrophenyliodoniumtetrakis(pentafluorophenyl)borat
- Bis(4-tert-butylphenyl)iodoniumtetrakis(pentafluorophenyl)borat
- Dinaphthyliodoniumtetrakis(pentafluorophenyl)borat
- Bis(4-dodecylphenyl)tetrakis(pentafluorophenyl)borat
- 4-Me-thylphenyl-4-isopropylphenyliodoniumtetrakis(pentafluorophenyl)borat

Unter α-Dicarbonylverbindungen des Bestandteils b) sind Verbindungen folgender Struktur zu verstehen: 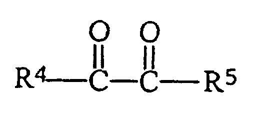 wobei R⁴ und R⁵ gleich oder verschieden, substituiert oder unsubstituiert, aliphatisch oder aromatisch sein können. R⁴ und R⁵ können zusammen Ringstrukturen bilden, die unsubstituiert oder substituiert mit aliphatischen, cycloaliphatischen, aromatischen, heteroaromatischen oder kondensierten aromatischen Resten sind. Bevorzugte α-Dicarbonylverbindungen sind Campherchinon, Benzil, 2,3-Butandion und 3,3,6,6-Tetramethylcyclohexandion, besonders bevorzugt ist Campherchinon.

Unter kationisch härtbaren Verbindungen des Bestandteils c) sind aliphatische oder aromatische Epoxide (Typ 1), cycloaliphatische Epoxide (Typ 2) oder Oxetane (Typ 3) mit folgenden Strukturen zu verstehen:

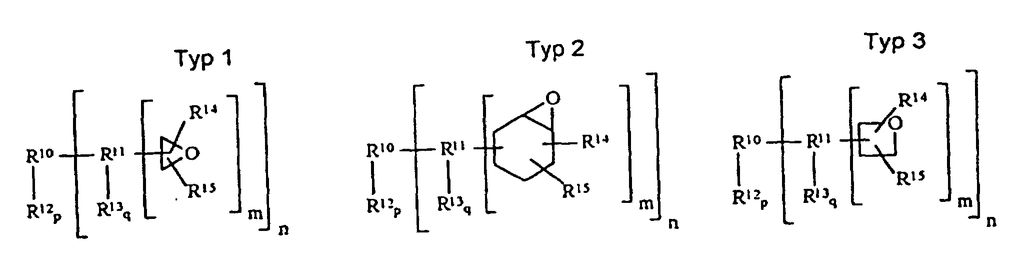

Es bedeuten:
- R¹⁰: einen aliphatischen, cycloaliphatischen oder aromatischen Rest mit 0 bis 22, vorzugsweise 0 bis 18 C-Atomen oder eine Kombination dieser Reste, wobei ein oder mehrere C-Atome durch O, C = O, -O(C = O)-, SiR₃ und/oder NR₂ ersetzt sein können und wobei R ein aliphatischer Rest mit 1 bis 7 C-Atomen ist, wobei ein oder mehrere C-Atome durch O, C = O und/oder -O(C = O)- ersetzt sein können,
- R¹¹: einen aliphatischen, cycloaliphatischen oder aromatischen Rest mit 1 bis 18, vorzugsweise 1 bis 15 C-Atomen oder eine Kombination dieser Reste, wobei ein oder mehrere C-Atome durch O, C =O, -O(C =O)-, SiR₃ und/oder NR₂ ersetzt sein können, wobei R ein aliphatischer Rest mit 1 bis 7 C-Atomen ist, bei dem ein oder mehrere C-Atome durch 0, C=O und/oder -O(C=O)- ersetzt sein können,
- R¹², R¹³, R¹⁴, R¹⁵: unabhängig voneinander ein H-Atom oder einen aliphatischen Rest mit 1 bis 9, vorzugsweise 1 bis 7 C-Atomen, wobei ein oder mehrere C-Atome durch 0, C =O, -O(C =O)-, SiR₃ und/oder NR₂ ersetzt sein können, wobei R ein aliphatischer Rest mit 1 bis 7 C-Atomen ist, bei dem ein oder mehrere C-Atome durch 0, C=O und/oder -O(C=O)- ersetzt sein können,
- n: 2 bis 7, vorzugsweise 2 bis 5, insbesondere 2 bis 4,
- m: 1 bis 10, vorzugsweise 1 bis 7, insbesondere 1 bis 5,
- p: 1 bis 5, vorzugsweise 1 bis 4, insbesondere 1 oder 2,
- q: 1 bis 5, vorzugsweise 1 bis 4, insbesondere 1 oder 2

Besonders geeignete Epoxide bzw. Oxetane gemäß Komponente c) sind
- Tetrakis(2,1-ethandiyl-3,4-epoxycyclohexyl)-1,3,5,7-tetramethylcyctotetrasiloxan, . 1,10-Decandiylbis(oxymethylen)bis(3-ethyloxetan),
- 1,3,5,7,9-Pentakis(2,1-ethandiyl-3,4-epoxycyclohexyl)-1,3,5,7,9-pentamethyl cyclopentasiloxan,
- Vinylcyclohexenoxid,
- Vinylcyclohexendioxid,
- 3,4-Epoxy-6-methylcyclohexylmethyl-3,4-epoxy-6-methylcyclohexencarboxylat,
- Bis(2,3-epoxycyclopentyl)ether
- 3,4-Epoxy-6-methylcyclohexylmethyladipat,
- 3,4-Epoxycyclohexyl-5,5-spiro-3,4-epoxycyclohexanmetadioxan,
- 1,4 Butandiyl-bisoxymethylenbis(3-ethyloxetan),
- 3,4-Epoxycyclohexylmethyl-3,4-epoxycyclohexancarboxylat,
- 1,1 ,3,3-Tetramethyl-l ,3-bis(2, l-ethandiyl-3,4-epoxycyclohexyl)disiloxan
- Bis-(3,4-Epoxycyclohexylmethyl)adipat.

Füllstoffe des Bestandteils d) können übliche dentale Füllstoffe, beispielsweise Quarz, gemahlene gegebenenfalls röntgenopake oder reaktive Gläser, Splitterpolymerisate, schwer lösliche Fluoride, wie CaF₂, YF₃ (EP-B- 0 238 025), Kieselgele sowie pyrogene Kieselsäure oder deren Granulate sein.

Ebenso können ein oder mehrere wasserlösliche anorganische komplexe Fluoride der allgemeinen Formel AₙMFₘ, worin A ein ein- oder mehrwertiges Kation, M ein Metall der II., III., IV. oder V. Haupt- oder Nebengruppe, n eine ganze Zahl von 1 bis 3 und m eine ganze Zahl von 3 bis 6 bedeuten (DE-A- 4 445 266), als fluoridabgebende Bestandteile enthalten sein.

Zum besseren Einbau in die Polymermatrix kann es von Vorteil sein, die Füllstoffe sowie gegebenenfalls die röntgenopaken Zusatzstoffe, wie YF₃, zu hydrophobieren. Übliche Hydrophobierungsmittel sind Silane, beispielsweise Glycidyloxypropyltrimethoxysilan. Die maximale Korngröße der anorganischen Füllstoffe beträgt vorzugsweise 20 µm, insbesondere 12 µm. Ganz besonders bevorzugt werden Füllstoffe mit einer mittleren Korngröße kleiner als 7 µm eingesetzt.

Die oxidierend wirkenden Zusatzstoffe von Bestandteil d) können organische oder anorganische Feststoffe oder Flüssigkeiten sein. Diese Zusatzstoffe beschleunigen die Initiierung und erhöhen den Polymerisationsgrad. Geeignete oxidierend wirkende Zusatzstoffe sind Hydroperoxide, wie Cumolhydroperoxid, Dialkylperoxide, wie Ditert.-butylperoxid, Diarylperoxide, wie Dibenzoylperoxid, Perester, wie tert.-Butylperbenzoat oder tert.-Butylisononanoat, anorganische Oxidationsmittel, wie Kaliumpersulfat, Natriumperborat, besonders bevorzugt Cumolhydroperoxid bzw. Kaliumpersulfat.

Unter den aromatischen Aminen des Bestandteils e) sind Verbindungen zu verstehen, die folgende Struktur aufweisen: 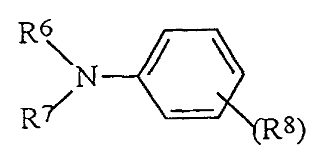 wobei R⁶, R⁷ und R⁸ gleich oder verschieden sind und unabhängig voneinander, ein H-Atom, einen aliphatischen, aromatischen oder araliphatischen Rest mit 1 bis 19, vorzugsweise 1 bis 7 C-Atomen, wobei ein oder mehrere C-Atome durch O, C = O, -O(C = O)-, SiR⁹₃ und/oder NR⁹₂ ersetzt sein können, wobei R⁹ ein aliphatischer Rest mit 1 bis 7 C-Atomen ist, bei dem ein oder mehrere C-Atome durch O, C = O und/oder -O(C = O)- ersetzt sein können, bedeuten und z Zahlenwerte von 1 bis 5 annehmen kann. R⁶ und R⁷ oder/und R⁶ und R⁸ können zusammen Ringstrukturen bilden, die unsubstituiert oder substituiert mit aliphatischen, cycloaliphatischen, aromatischen, heteroaromatischen oder kondensierten aromatischen Resten sind.

Bevorzugte Amine sind Dimethylanilin, Diethylanilin, Ethyl-4-dimethyl-aminobenzoat, 2-Butoxyethyl-4-dimethylaminobenzoat, 2-Ethylhexyl-4-dimethylaminobenzoat, 4-Dimethylaminobenzaldehyd und seine Derivate, wie Benzaldoxime, Azomethine, Benzylidenanilin, Hydrobenzamid, Benzoine, Benzile, Hydrobenzoine, Benzoesäurebenzylester.

Die erfindungsgemäßen Zusammensetzungen werden durch Zusammenmischen der einzelnen Bestandteile hergestellt. Die Reihenfolge der Zugabe ist nicht kritisch. Vorzugsweise wird die Epoxidgruppen- und/oder Oxetangruppen-haltige Verbindung vorgelegt und in diese die Diaryliodoniumverbindung, die α-Dicarbonylverbindung und das aromatische Amin eingerührt. Anschließend werden der Füllstoff und die anderen Modifizierungsmittel eingeknetet.

Die erfindungsgemäßen Zusammensetzungen eignen sich zur lichtinduzierten kationischen Polymerisation auf der Grundlage von Epoxidgruppen- und bzw. oder Oxetangruppen enthaltenden Verbindungen. Das Initiatorsystem ist geeignet, gefüllte Zusammensetzungen auszuhärten. Einen besonderen Vorteil bieten die Zusammensetzungen bei der dentalen Anwendung. Die polymerisierbaren Zubereitungen haben überraschend kurze Aushärtungszeiten, wobei diese Aushärtungszeiten durch Art und Konzentration zusätzlicher Aktivatoren, beispielsweise oxidierend wirkender Zusatzstoffe, sehr genau eingestellt werden können.

Die erfindungsgemäßen Zusammensetzungen eignen sich für das Verkleben, Vergießen und Beschichten von Substraten sowie für Dentalmassen.

Ein sehr wesentlicher Vorteil der erfindungsgemäßen Zusammensetzungen ist der außerordentlich gute ästhetische Eindruck der ausgehärteten Massen. Die Polymerisate sind zahnfarben einstellbar und dadurch für die dentale Anwendung besonders geeignet. Durch die hohe Transparenz der Polymerisate wird eine außergewöhnliche Härtungstiefe erreicht.

Die Erfindung wird anhand der folgenden Beispiele weiter erläutert:

### Beispiele

Durch Mischen der in Tabelle 1 beschriebenen Verbindungen wurden die nicht gefüllten, kationisch härtbaren einkomponentigen Zusammensetzungen gemäß den Beispielen 1 bis 7 hergestellt. Diese Zusammensetzungen stellen die Harzmatrix für daraus hergestellte gefüllte Zusammensetzungen (Composite) gemäß den Beispielen 8 und 9 dar (Tabelle 2)

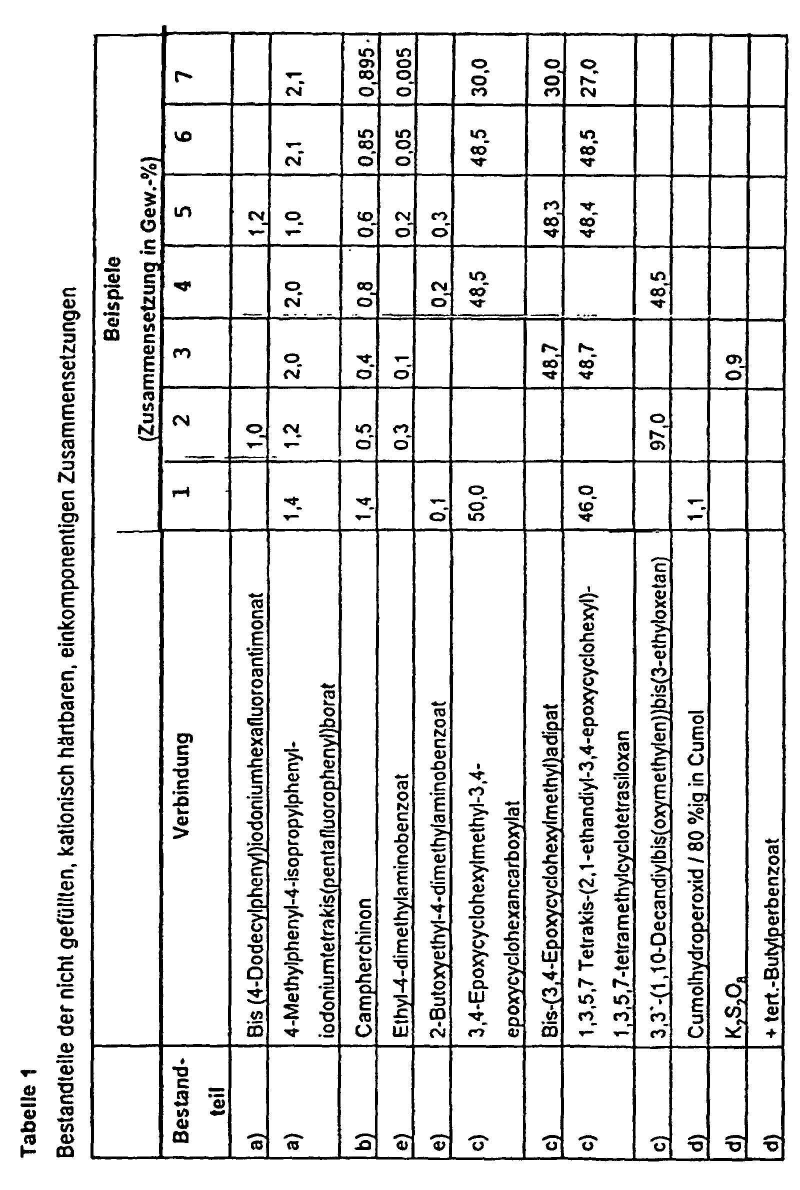

Alle Zusammensetzungen gemäß den Beispielen 1 bis 7 härteten bei Bestrahlung mit einer Lampe (Lichtgerät Elipar II, ESPE Dental-Medizin GmbH & Co. KG Deutschland), die sichtbares Licht im Wellenlängenbereich von 400 bis 500 nm erzeugt, innerhalb von 20 Sekunden aus. Die erhaltenen Polymerisate waren transparent und wiesen hohe Festigkeiten auf.

Die in Tabelle 2 charakterisierten Komposit-Zusammensetzung gemäß den Beispielen 8 und 9 härteten bei Bestrahlung mit der beschriebenen Lampe innerhalb von 40 Sekunden aus.

**Tabelle 2**

| Zusammensetzung der gefüllten, kationisch härtbaren einkomponentigen Zubereitungen | | | |
|---|---|---|---|
| Beispiel-Nr. | Nicht gefüllte Zusammensetzung | | Füllstoff |
| | Zusammensetzung entsprechend Beispiel Nr. | Gew.-% | Quarz^{a)} Gew.-% |
| 8 | 3 | 30 | 70 |
| 9 | 7 | 40 | 60 |

| | | | |
|---|---|---|---|
| a) Quarz, mittlere Korngröße 0,9*µ*m wurde mit 5 % Glycidyloxypropyltrimethoxysilan silanisiert | | | |

Die erhaltenen Polymerisate waren farblos bis zahnfarben.

Tabelle 3 enthält die Ergebnisse der Ermittlung wesentlicher Eigenschaften, die unter Verwendung der Komposit-Zusammensetzungen gemäß den Beispielen 8 und 9 erreicht wurden

**Tabelle 3**

| Eigenschaften der ausgehärteten Massen der Beispiele 8 und 9. | | |
|---|---|---|
| **Beispiel** | **Biegefestigkeit ISO 4049** | **Druckfestigkeit** |
| 8 | 110 MPa | 381 MPa |
| 9 | 95 MPa | 352 MPa |

Die Beispiele belegen, daß mit den erfindungsgemäßen Zusammensetzungen ausgehärtete Massen erzielbar sind, sie sich auf Grund der nur geringen Eigenfarbe und der sehr guten mechanischen Eigenschaften für dentale Anwendungen und insbesondere für Füllungsmaterialien und Befestigungszemente hervorragend eignen.

## Patentansprüche

1. Mit sichtbarem Licht kationisch härtende Zusammensetzung, enthaltend
(a) 0,01 bis 8 Gew.-% mindestens einer Diaryliodoniumverbindung, mit folgender Struktur:
[((R¹)ₐAr¹)-I-Ar²(R²)_{b})]⁺ Y⁻
wobei bedeuten:
Ar¹ , Ar² unabhängig voneinander verschiedene, substituierte oder unsubstituierte, kondensierte oder nichtkondensierte aromatische Systeme mit 4 bis 20 C-Atomen, vorzugsweise Phenyl, Tolyl, Cumyl, Anisyl, Chlorphenyl, Nitrophenyl, Naphtyl, Thienyl, Furanyl und Pyrazolyl
R¹, R² unabhängig voneinander ein H-Atom, einen aliphatischen Rest mit 1 bis 19, vorzugsweise 1 bis 9 C-Atomen, wobei ein oder mehrere C-Atome durch O, C = O, -O(C = O)-, F, Cl, Br, SiR³₃ und/oder NR³₂ ersetzt sein können, wobei R³ ein aliphatischer Rest mit 1 bis 7 C-Atomen ist, bei dem ein oder mehrere C-Atome durch O, C = O und/oder -O(C = O)- ersetzt sein können, wobei die Aromaten Ar¹ und Ar² über R¹ und/oder R² miteinander verbunden sein können,
a und b unabhängig voneinander eine ganze Zahl von 1 bis 5, und
Y⁻ ein wenig nukleophiles Anion der Formel
KₓL_{y}⁻
worin bedeuten:
K ein Element der III., V. oder VII. Hauptgruppe, vorzugsweise B, Al, P, Sb, As oder I
x eine Zahl von 1 bis 4
L voneinander unabhängige aromatische, araliphatische oder cycloaliphatische Reste mit bis zu 25 C-Atomen, bei denen ein oder mehrere C-Atome durch F, Cl, Br oder I ersetzt sein können, und
y eine Zahl von 0 bis 6.
(b) 0,01 bis 8 Gew.-% mindestens einer α-Dicarbonylverbindung,
(c) 10,0 bis 99,9 Gew.-% mindestens einer Epoxidgruppen- und/oder Oxetangruppen-enthaltenden Verbindung,
(d) O bis 85 Gew.-% an Modifikatoren, wie Füllstoffen, Farbstoffen, Pigmenten, Fließverbesserern, Thixotropiemitteln, polymeren Verdickern, oxidierend wirkenden Zusatzstoffen, Stabilisatoren und Verzögerern, und
(e) 0,001 bis 5 Gew.-% mindestens eines aromatischen Amins.

2. Zusammensetzung nach Anspruch 1, **dadurch gekennzeichnet, daß** sie die Bestandteile
(a) in einer Menge von 0,1 bis 5 Gew.-%,
(b) in einer Menge von 0,1 bis 5 Gew.-% und
(e) in einer Menge von 0,01 bis 3 Gew.-%
enthält.

3. Zusammensetzung nach Anspruch 1, **dadurch gekennzeichnet, daß** Y⁻ B(C₆F₅)₄⁻ ist.

4. Zusammensetzung nach den Ansprüchen 1 bis 3, **dadurch gekennzeichnet, daß** die α-Dicarbonylverbindungen des Bestandteils (b) folgende Struktur aufweisen: 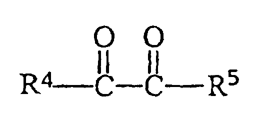 in welcher R⁴ und R⁵ unabhängig voneinander substituiert oder unsubstituiert, aliphatisch oder aromatisch sein können und R⁴ und R⁵ zusammen Ringstrukturen bilden können, die unsubstituiert oder substituiert mit aliphatischen, cycloaliphatischen, aromatischen, heteroaromatischen oder kondensierten aromatischen Resten sind.

5. Zusammensetzung nach Anspruch 4, **dadurch gekennzeichnet, daß** sie als Bestandteil (b) Campherchinon, Benzil, 2,3-Butandion und/oder 3,3,6,6-Tetramethylcyclohexandion, vorzugsweise Campherchinon, enthält.

6. Zusammensetzung nach den Ansprüchen 1 bis 5, **dadurch gekennzeichnet, daß** die aromatischen Amine des Bestandteil (e) die folgende Struktur besitzen: 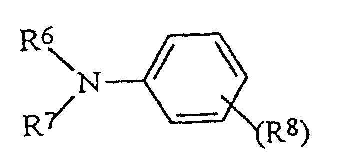 in der R⁶, R⁷ und R8 unabhängig voneinander ein H-Atom, einen aliphatischen, aromatischen oder araliphatischen Rest mit 1 bis 19, vorzugsweise 1 bis 7 C-Atomen bedeuten, wobei ein oder mehrere C-Atome durch O, C=O, -O(C = O)-, SiR⁹₃ und/oder NR⁹₂ ersetzt sein können und R⁹ ein aliphatischer Rest mit 1 bis 7 C-Atomen ist, bei dem ein oder mehrere C-Atome durch O, C=O und/oder -O(C =O)-ersetzt sein können, und z Zahlenwerte von 1 bis 5 annehmen kann und R⁶ und R⁷ oder/und R⁶ und R⁸ zusammen Ringstrukturen bilden können die unsubstituiert oder mit aliphatischen, cycloaliphatischen, aromatischen, heteroaromatischen oder kondensierten aromatischen Resten substituiert sein können.

7. Zusammensetzung nach Anspruch 6, **dadurch gekennzeichnet, daß** sie als Bestandteil (c) Dimethylanilin, Diethylanilin, Ethyl-4-dimethyl-aminobenzoat, 2-Butoxyethyl-4-dimethylaminobenzoat, 2-Ethylhexyl-4-dimethylaminobenzoat, 4-Dimethylaminobenzaldehyd, Benzaldoxime, Azomethine, Benzylidenanilin, Hydrobenzamid, Benzoine, Benzile, Hydrobenzoine und/oder Benzoesäurebenzylester, vorzugsweise Ethyl-4-dimethyl-aminobenzoat, 2-Butoxyethyl-4-dimethylaminobenzoat, 2-Ethylhexyl-4-dimethylaminobenzoat enthält.

8. Zusammensetzung nach den Ansprüchen 1 bis 7, **dadurch gekennzeichnet, daß** die Epoxidgruppen und/oder Oxetangruppen-enthaltenden Verbindungen des Bestandteils (c) folgende Struktur aufweisen: 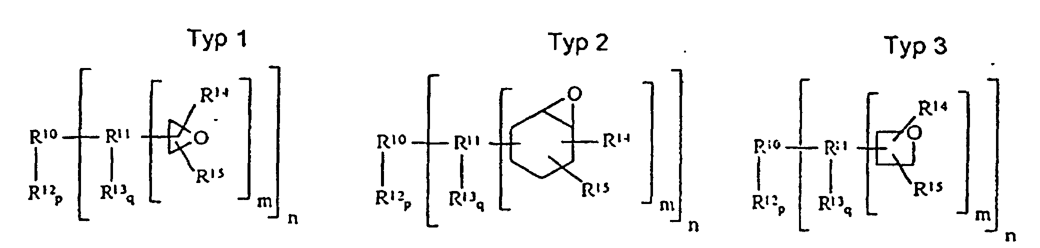 wobei bedeuten:
R¹⁰ einen aliphatischen, cycloaliphatischen oder aromatischen Rest mit 0 bis 22, vorzugsweise 0 bis 18 C-Atomen oder eine Kombination dieser Reste, wobei ein oder mehrere C-Atome durch O, C=O, -O(C=O)-, SiR₃ und/oder NR₃ ersetzt sein können und wobei R ein aliphatischer Rest mit 1 bis 7 C-Atomen ist, wobei ein oder mehrere C-Atome durch O, C = O und/oder -O(C = O)- ersetzt sein können,
R¹¹ einen aliphatischen, cycloaliphatischen oder aromatischen Rest mit 1 bis 18, vorzugsweise 1 bis 15 C-Atomen oder eine Kombination dieser Reste, wobei ein oder mehrere C-Atome durch O, C=O, -O(C=O)-, SiR₃ und/oder NR₂ ersetzt sein können, wobei R ein aliphatischer Rest mit 1 bis 7 C-Atomen ist, bei dem ein oder mehrere C-Atome durch O, C=O und/oder -O(C = O)- ersetzt sein können,
R¹², R¹³, R¹⁴, R¹⁵ unabhängig voneinander ein H-Atom oder einen aliphatischen Rest mit 1 bis 9, vorzugsweise 1 bis 7 C-Atomen, wobei ein oder mehrere C-Atome durch O, C=O, -O(C =O)-, SiR₃ und/oder NR₂ ersetzt sein können, wobei R ein aliphatischer Rest mit 1 bis 7 C-Atomen ist, bei dem ein oder mehrere C-Atome durch O, C=O und/oder -O(C=O)- ersetzt sein können,
n 2 bis 7, vorzugsweise 2 bis 5, insbesondere 2 bis 4,
m 1 bis 10, vorzugsweise 1 bis 7, insbesondere 1 bis 5,
p 1 bis 5, vorzugsweise 1 bis 4, insbesondere 1 oder 2,
q 1 bis 5, vorzugsweise 1 bis 4, insbesondere 1 oder 2.

9. Zusammensetzung nach Anspruch 8, **dadurch gekennzeichnet, daß** sie als Komponente (c) 1,3,5,7-Tetrakis-(2,1-ethandiyl-3,4-epoxycyclohexyl)-1,3,5,7-tetramethylcyclotetrasiloxan, 1,10-Decandiylbis(oxymethylen)bis(3-ethyloxetan), 1,3,5,7,9-Pentakis(2,1-ethandiyl-3,4-epoxycyclohexyl)-1,3,5,7,9-pentamethylcyclopentasiloxan, Vinylcyclohexenoxid, Vinylcyclohexendioxid, 3,4-Epoxy-6-methylcyclohexylmethyl-3,4-epoxy-6-methylcyclohexencarboxylat, Bis (2,3-epoxycyclopentyl) ether; 3,4-Epoxy-6-methylcyclohexylmethyl)adipat, (3,4-Epoxycyclohexyl-5,5-spiro-3,4-epoxy (cyclohexanmetadioxan), 1,4 Butandiyl-bis(oxymethylen) bis (3-ethyloxetan), 3,4-Epoxycyclohexylmethyl-3,4-epoxycyclohexancarboxylat, 1,1,3,3-Tetramethyl-1,3-bis(2,1-ethandiyl-3,4-epoxycyclohexyl)disiloxan und/oder Bis-(3,4-Epoxycyclohexylmethyl)adipat enthält.

10. Zusammensetzung nach den Ansprüchen 1 bis 9, **dadurch gekennzeichnet, daß** sie als oxidierend wirkende Zusatzstoffe des Bestandteils (d) Cumolhydroperoxid, Di-tert.-butylperoxid, Diarylperoxide, Dibenzoylperoxid, tert.-Butylperbenzoat, tert.-Butylisononanoat, Kaliumpersulfat und/oder Natriumperborat enthält.

11. Zusammensetzung nach den Ansprüchen 1 bis 9, **dadurch gekennzeichnet, daß** sie als Füllstoffe des Bestandteils (d) Quarz, gemahlene gegebenenfalls röntgenopake oder reaktive Gläser, Splitterpolymerisate, schwer lösliche Fluoride, wie CaF₂, YF₃, Kieselgele und/oder pyrogene Kieselsäure oder deren Granulate enthält.

12. Verwendung der Zusammensetzungen nach den Ansprüchen 1 bis 11 für das Verkleben, Vergießen und Beschichten von Substraten.

13. Verwendung der Zusammensetzungen nach den Ansprüchen 1 bis 11 zur Herstellung von Dentalmassen, insbesondere zur Herstellung von Zahnfüllungsmaterialien, Bondingmaterialien, Füllungs- und Befestigungszementen, Provisorienkunststoffen, Verblendmaterialien, Versiegelungsmaterialien, Prothesenzähnen, Kunststoffen zur Anfertigung von totalen oder partiellen Prothesen.

## Claims

1. Composition cationically curing with visible light, comprising
a) 0.01 to 8% by weight of at least one diaryliodonium compound with the following structure:
[((R¹)ₐAr¹)-I-(Ar²(R²)_{b})]⁺ Y⁻
in which:
Ar¹ and Ar² represent, independently of one another, different, substituted or unsubstituted, condensed or noncondensed aromatic systems with 4 to 20 carbon atoms, preferably phenyl, tolyl, cumyl, anisyl, chlorophenyl, nitrophenyl, napthyl, thienyl, furanyl and pyrazolyl,
R¹ and R² represent, independently of one another, a hydrogen atom, an aliphatic radical with 1 to 19, preferably 1 to 9, carbon atoms in which one or more carbon atoms can be replaced by O, C=O, -O(C=O)-, F, Cl, Br or SiR³₃ and/or NR³₂, in which R³ is an aliphatic radical with 1 to 7 carbon atoms in which one or more carbon atoms can be replaced by 0, C=O and/or -O(C=O)-, in which the aromatic units Ar¹ and Ar² can be bonded to one another via R¹ and/or R²,
a and b represent, independently of one another, an integer from 1 to 5, and
Y⁻ represents a weakly nucleophilic anion of the formula
KₓL_{y}⁻
in which:
K represents an element from the IIIrd, Vth or VIIth main group, preferably B, Al, P, Sb, As or I,
x represents a number from 1 to 4,
L represents, independently of one another, aromatic, araliphatic or cycloaliphatic radicals with up to 25 carbon atoms in which one or more carbon atoms can be replaced by F, Cl, Br or I, and
y represents a number from 0 to 6,
b) 0.01 to 8% by weight of at least one α-dicarbonyl compound,
c) 10.0 to 99.9% by weight of at least one compound comprising epoxide groups and/or oxetane groups,
d) 0 to 85% by weight of modifiers, such as fillers, dyes, pigments, flow improvers, thixotropic agents, polymeric thickeners, additives with an oxidizing effect, stabilizers and inhibitors, and
e) 0.001 to 5% by weight of at least one aromatic amine.

2. Composition according to Claim 1, **characterized in that** it comprises the constituents
(a) in an amount of 0.1 to 5% by weight,
(b) in an amount of 0.1 to 5% by weight and
(e) in an amount of 0.01 to 3% by weight.

3. Composition according to Claim 1, **characterized in that** Y⁻ is B(C₆F₅)₄⁻.

4. Composition according to Claims 1 to 3, **characterized in that** the α-dicarbonyl compounds of the constituent (b) exhibit the following structure: 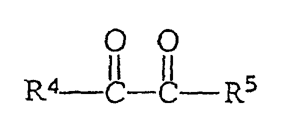 in which R⁴ and R⁵ can be, independently of one another, substituted or unsubstituted, aliphatic or aromatic, and R⁴ and R⁵ can together form ring structures which are unsubstituted or substituted with aliphatic, cycloaliphatic, aromatic, heteroaromatic or condensed aromatic radicals.

5. Composition according to Claim 4, **characterized in that** it comprises camphorquinone, benzil, 2,3-butanedione and/or 3,3,6,6-tetramethylcyclohexanedione, preferably camphorquinone, as constituent (b).

6. Composition according to Claims 1 to 5, **characterized in that** the aromatic amines of the constituent (e) have the following structure: 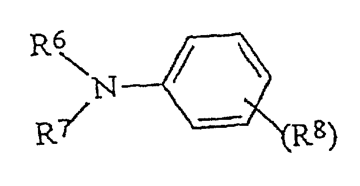 in which R⁶, R⁷ and R⁸ represent, independently of one another, a hydrogen atom or an aliphatic, aromatic or araliphatic radical with 1 to 19, preferably 1 to 7, carbon atoms in which one or more carbon atoms can be replaced by O, C=O, -O(C=O)-, SiR⁹₃ and/or NR⁹₂ and R⁹ is an aliphatic radical with 1 to 7 carbon atoms in which one or more carbon atoms can be replaced by O, C=O and/or -O(C=O)-, and z can assume numerical values from 1 to 5, and R⁶ and R⁷ and/or R⁶ and R⁸ can together form ring structures which can be unsubstituted or substituted with aliphatic, cycloaliphatic, aromatic, heteroaromatic or condensed aromatic radicals.

7. Composition according to Claim 6, **characterized in that** it comprises dimethylaniline, diethylaniline, ethyl 4-dimethylaminobenzoate, 2-butoxyethyl 4-dimethylaminobenzoate, 2-ethylhexyl 4-dimethylaminobenzoate, 4-dimethylaminobenzaldehyde, benzaldoximes, azomethines, benzylideneaniline, hydrobenzamide, benzoins, benzils, hydrobenzoins and/or benzyl benzoates, preferably ethyl 4-dimethylaminobenzoate, 2-butoxyethyl 4-dimethylaminobenzoate or 2-ethylhexyl 4-dimethylaminobenzoate, as constituent (c).

8. Composition according to Claims 1 to 7, **characterized in that** the compounds comprising epoxide groups and/or oxetane groups of the constituent (c) exhibit the following structure: 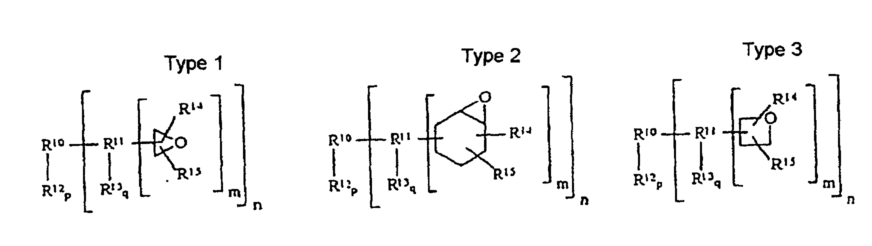 where
R¹⁰ represents an aliphatic, cycloaliphatic or aromatic radical with 0 to 22, preferably 0 to 18, carbon atoms or a combination of these radicals in which one or more carbon atoms can be replaced by 0, C=O, -O(C=O)-, SiR₃ and/or NR₃ and in which R is an aliphatic radical with 1 to 7 carbon atoms in which one or more carbon atoms can be replaced by O, C=O and/or -O(C=O)-,
R¹¹ represents an aliphatic, cycloaliphatic or aromatic radical with 1 to 18, preferably 1 to 15, carbon atoms or a combination of these radicals in which one or more carbon atoms can be replaced by O, C=O, -O(C=O)-, SiR₃ and/or NR₂, in which R is an aliphatic radical with 1 to 7 carbon atoms in which one or more carbon atoms can be replaced by O, C=O and/or -O(C=O)-,
R¹², R¹³, R¹⁴ and R¹⁵ represent, independently of one another, a hydrogen atom or an aliphatic radical with 1 to 9, preferably 1 to 7, carbon atoms in which one or more carbon atoms can be replaced by O, C=O, -O(C=O)-, SiR₃ and/or NR₂, in which R is an aliphatic radical with 1 to 7 carbon atoms in which one or more carbon atoms can be replaced by 0, C=O and/or -O(C=O)-,
n represents 2 to 7, preferably 2 to 5, in particular 2 to 4,
m represents 1 to 10, preferably 1 to 7, in particular 1 to 5,
p represents 1 to 5, preferably 1 to 4, in particular 1 or 2,
q represents 1 to 5, preferably 1 to 4, in particular 1 or 2.

9. Composition according to Claim 8, **characterized in that** it comprises as component (c) 1,3,5,7-tetrakis(2,1-ethanediyl-3,4-epoxycyclohexyl)-1,3,5,7-tetramethylcyclotetrasiloxane, 1,10-decanediylbis(oxymethylene)bis(3-ethyloxetane), 1,3,5,7,9-pentakis(2,1-ethanediyl-3,4-epoxycyclohexyl)-1,3,5,7,9-pentamethylcyclopentasiloxane, vinylcyclohexene oxide, vinylcyclohexene dioxide, 3,4-epoxy-6-methylcyclohexylmethyl 3,4-epoxy-6-methylcyclohexenecarboxylate, bis(2,3-epoxycyclopentyl) ether; 3,4-epoxy-6-methylcyclohexylmethyl adipate, 3,4-epoxycyclohexyl-5,5-spiro-3,4-epoxy(cyclohexane-meta-dioxane), 1,4-butanediylbisoxymethylenebis-(3-ethyloxetane), 3,4-epoxycyclohexylmethyl 3,4-epoxycyclohexanecarboxylate, 1,1,3,3-tetramethyl-1,3-bis-(2,1-ethanediyl-3,4-epoxycyclohexyl)disiloxane, and/or bis(3,4-epoxycyclohexylmethyl) adipate.

10. Composition according to Claims 1 to 9, **characterized in that** it comprises, as additives with an oxidizing effect of constituent (d), cumene hydroperoxide, di-tert-butyl peroxide, diaryl peroxides, dibenzoyl peroxide, tert-butyl perbenzoate, tert-butyl isononanoate, potassium persulphate and/or sodium perborate.

11. Composition according to Claims 1 to 9, **characterized in that** it comprises as fillers of the constituent (d) quartz, ground glass, if appropriate opaque to X-rays or reactive, fragmented polymers, sparingly soluble fluorides, such as CaF₂ or YF₃, silica gels and/or pyrogenic silica or their granules.

12. Use of the compositions according to Claims 1 to 11 for the cementing, embedding and coating of substrates.

13. Use of the compositions according to Claims 1 to 11 for producing dental compositions, especially for producing dental filling materials, bonding materials, filling and fixing cements, provisional restoration plastics, facing materials, sealing materials, prosthetic teeth, plastics for preparing total or partial prostheses.

## Revendications

1. Composition durcissable par voie cationique avec de la lumière visible, contenant
a) 0,01 à 8 % en poids d'au moins un composé de diaryliodonium, avec la structure suivante :
[((R¹)ₐAr¹)-I-(Ar²(R²)_{b})]⁺ Y⁻
dans laquelle :
Ar¹, Ar² représentent, indépendamment l'un de l'autre, des systèmes aromatiques différents substitués ou non substitués, condensés ou non condensés, ayant de 4 à 20 atomes de carbone, de préférence le phényle, tolyle, cumyle, anisyle, chlorophényle, nitrophényle, naphtyle, thiényle, furanyle et pyrazolyle,
R¹, R² représentent, indépendamment l'un de l'autre, un atome H, un radical aliphatique avec de 1 à 19, de préférence de 1 à 9 atomes de carbone, dans lequel un ou plusieurs atomes de carbone peuvent être remplacés par O, C=O, -O(C=O)-, F, Cl, Br, SiR³₃ et/ou NR³₂, où R³ est un radical aliphatique avec de 1 à 7 atomes de carbone, dans lequel un ou plusieurs atomes de carbone peuvent être remplacés par O, C=O et/ou -O(C=O)-, les groupes aromatiques Ar¹ et Ar² pouvant être liés entre eux par R¹ et/ou R²,
a et b représentent, indépendamment l'un de l'autre, un nombre entier de 1 à 5, et
Y⁻ représente un anion peu nucléophile de formule
KₓL_{y}⁻
dans laquelle :
K représente un élément du groupe principal III, V ou VII, de préférence B, Al, P, Sb, As ou I
x est un nombre de 1 à 4,
L représentent, indépendamment les uns des autres, des radicaux aromatiques, araliphatiques ou cycloaliphatiques ayant jusqu'à 25 atomes de carbone, dans lesquels un ou plusieurs atomes de carbone peuvent être remplacés par F, Cl, Br ou I, et
y est un nombre de 0 à 6,
b) 0,01 à 8 % en poids d'au moins un composé α-dicarbonylique,
c) 10,0 à 99,9 % en poids d'au moins un composé contenant des groupes époxyde et/ou des groupes oxétane,
d) 0 à 85 % en poids de modificateurs, comme des charges, des colorants, des pigments, des agents d'amélioration d'écoulement, des agents de thixotropie, des épaississants polymères, des additifs agissant comme oxydants, des stabilisants et des retardateurs, et
e) 0,001 à 5 % en poids d'au moins une amine aromatique.

2. Composition selon la revendication 1, **caractérisée en ce qu'**elle contient les constituants
a) en une quantité de 0,1 à 5 % en poids,
b) en une quantité de 0,1 à 5 % en poids, et
e) en une quantité de 0,01 à 3 % en poids.

3. Composition selon la revendication 1, **caractérisée en ce que** Y⁻ est B(C₆F₅)₄⁻.

4. Composition selon les revendications 1 à 3, **caractérisée en ce que** les composés α-dicarbonyliques du constituant (b) présentent la structure suivante : 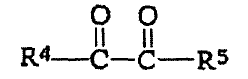 dans laquelle R⁴ et R⁵ indépendamment l'un de l'autre peuvent être substitués ou non substitués, aliphatiques ou aromatiques et R⁴ et R⁵ peuvent former ensemble des structures cycliques, qui sont substituées ou non substituées avec des radicaux aliphatiques, cycloaliphatiques, aromatiques, hétéroaromatiques ou aromatiques condensés.

5. Composition selon la revendication 4, **caractérisée en ce qu'**elle contient comme constituant (b) la camphroquinone, le benzile, la 2,3-butanedione et/ou la 3,3,6,6-tétraméthylcyclohexanedione, de préférence la camphroquinone.

6. Composition selon les revendications 1 à 5, **caractérisée en ce que** les amines aromatiques du constituant (e) possèdent la structure suivante : 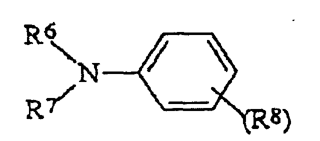 dans laquelle R⁶, R⁷ et R⁸ représentent indépendamment les uns des autres un atome H, un radical aliphatique, aromatique ou araliphatique avec de 1 à 19, de préférence 1 à 7 atomes de carbone, dans lequel un ou plusieurs atomes de carbone peuvent être remplacés par O, C=O, -O(C=O)-, SiR⁹₃ et/ou NR⁹₂, et R⁹ est un groupe aliphatique avec de 1 à 7 atomes de carbone, dans lequel un ou plusieurs atomes de carbone peuvent être remplacés par O, C=O et/ou -O(C=O)-, et z peut prendre des valeurs numériques de 1 à 5 et R⁶ et R⁷ et/ou R⁶ et R⁸ peuvent former ensemble des structures cycliques, qui peuvent être non substituées ou substituées avec des radicaux aliphatiques, cycloaliphatiques, aromatiques, hétéro-aromatiques ou aromatiques condensés.

7. Composition selon la revendication 6, **caractérisée en ce qu'**elle contient comme constituant (c) la diméthylaniline, la diéthylaniline, le 4-diméthylaminobenzoate d'éthyle, le 4-diméthylaminobenzoate de 2-butoxyéthyle, le 4-diméthylaminobenzoate de 2-éthylhexyle, le 4-diméthylaminobenzaldéhyde, les benzaldoximes, les azométhines, la benzilidèneaniline, l'hydrobenzamide, la benzoïne, le benzile, l'hydrobenzoïne et/ou l'ester benzylique d'acide benzoïque, de préférence le 4-diméthylaminobenzoate d'éthyle, le 4-diméthylaminobenzoate de 2-butoxyéthyle, le 4-diméthylaminobenzoate de 2-éthylhexyle.

8. Composition selon les revendications 1 à 7, **caractérisée en ce que** les composés du constituant (c) contenant des groupes époxyde et/ou des groupes oxétane présentent la structure suivante : 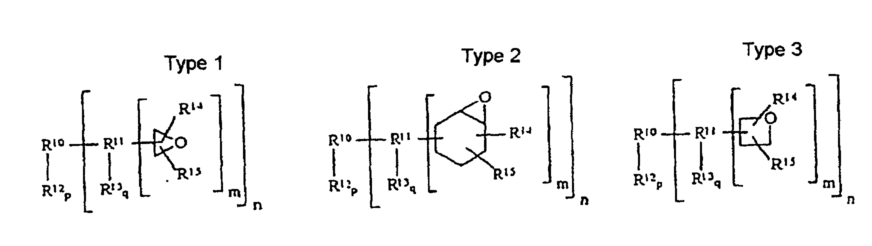 dans laquelle :
R¹⁰ représente un radical aliphatique, cycloaliphatique ou aromatique avec de 0 à 22, de préférence de 0 à 18 atomes de carbone ou une combinaison de ces radicaux, dans lequel un ou plusieurs atomes de carbone peuvent être remplacés par O, C=O, -O(C=O)-, SiR₃ et/ou NR₃, où R est un radical aliphatique avec de 1 à 7 atomes de carbone, dans lequel un ou plusieurs atomes de carbone peuvent être remplacés par O, C=O et/ou -O(C-O)-,
R¹¹ représente un radical aliphatique, cycloaliphatique ou aromatique avec de 1 à 18, de préférence de 1 à 15 atomes de carbone ou une combinaison de ces radicaux, dans lequel un ou plusieurs atomes de carbone peuvent être remplacés par O, C=O, -O(C=O)-, SiR₃ et/ou NR₂, où R est un radical aliphatique avec de 1 à 7 atomes de carbone, dans lequel un ou plusieurs atomes de carbone peuvent être remplacés par O, C=O et/ou -O(C=O)-,
R¹², R¹³, R¹⁴, R¹⁵ représentent, indépendamment les uns des autres, un atome H ou un radical aliphatique avec de 1 à 9, de préférence de 1 à 7 atomes de carbone, dans lesquels un ou plusieurs atomes C peuvent être remplacés par 0, C=O, -O(C=O)-, SiR₃ et/ou NR₂, où R est un radical aliphatique avec de 1 à 7 atomes de carbone, dans lequel un ou plusieurs atomes de carbone peuvent être remplacés par O, C=O et/ou -O(C=O)-,
n représente 2 à 7, de préférence 2 à 5, en particulier 2 à 4,
m représente 1 à 10, de préférence 1 à 7, en particulier 1 à 5,
p représente 1 à 5, de préférence 1 à 4, en particulier 1 ou 2,
q représente 1 à 5, de préférence 1 à 4, en particulier 1 ou 2.

9. Composition selon la revendication 8, **caractérisée en ce qu'**elle contient comme composant (c) le 1,3,5,7-tétrakis-(2,1-éthanediyl-3,4-époxycyclohexyl)-1,3,5,7-tétraméthylcyclotétrasiloxane, le 1,10-décanediylbis(oxyméthylène)bis(3-éthyloxétane), le 1,3,5,7,9-pentakis(2,1-éthanediyl-3,4-époxycyclohexyl)-1,3,5,7,9-pentaméthylcyclopentasiloxane, l'oxyde de vinylcyclohexène, le dioxyde de vinylcyclohexène, le 3,4-époxy-6-méthylcyclohexylméthyl-3,4-époxy-6-méthylcyclohexène-carboxylate, le bis(2,3-époxycyclopentyl)éther, le 3,4-époxy-6-méthylcyclohexylméthyladipate, le (3,4-époxycyclohexyl-5,5-spiro-3,4-époxy (cyclohexanemétadioxane), le 1,4-butanediylbis(oxyméthylène)bis(3-éthyloxétane), le 3,4-époxycyclohexylméthyl-3,4-époxycyclohexanecarboxylate, le 1,1,3,3-tétraméthyl-1,3-bis(2,1-éthanediyl-3,4-époxycyclohexyl)disiloxane et/ou le bis(3,4-époxycyclohexyl-méthyl)adipate.

10. Composition selon les revendications 1 à 9, **caractérisée en ce qu'**elle contient comme additifs agissant comme oxydants du constituant (d) l'hydroperoxyde de cumène, le peroxyde de di-tert.-butyle, des peroxydes de diaryle, le peroxyde de dibenzoyle, le perbenzoate de tert.-butyle, l'isononanoate de tert.-butyle, le persulfate de potassium et/ou le perborate de sodium.

11. Composition selon les revendications 1 à 9, **caractérisée en ce qu'**elle contient comme charges du constituant (d) du quartz, des verres broyés éventuellement opaques aux rayons X ou réactifs, des polymérisats en copeaux, des fluorures peu solubles, comme CaF₂, YF₃, des gels de silice et/ou de silice pyrogène ou leurs granulés.

12. Utilisation des compositions selon les revendications 1 à 11 pour le collage, le coulage et le revêtement de substrats.

13. Utilisation des compositions selon les revendications 1 à 11 pour la préparation de pâtes dentaires, en particulier pour la préparation de matériaux de plombage dentaire, de matériaux de collage, de ciments de plombage et de renforcement, de matières synthétiques pour pansements, de matériaux de mélange, de matériaux de scellement, de dents de prothèse, de matières synthétiques pour la fabrication de prothèses totales ou partielles.
